# EUROPEAN PATENT APPLICATION

(11) **EP 4 140 451 A1**
(43) Date of publication of application: **01.03.2023**
(21) Application number: 20932333.6
(22) Date of filing: 15.05.2020
(51) Int. Cl.: A61F 2/38

(54) **ANATOMICAL KNEE JOINT PROSTHESIS**

(30) Priority: 23.04.2020 CN 202010324496
(71) Applicant: Beijing Chunlizhengda Medical Instruments Co., Ltd, Beijing 101112 (CN)
(72) Inventor: XIE, Fengbao, Beijing 100000 (CN); SHI, Chunbao, Beijing 100000 (CN); HAN, Chuan, Beijing 100000 (CN)
(74) Representative: Dr. Gassner & Partner mbB
(86) International application number: PCT/CN2020/090638
(87) International publication number: WO 2021/212580

(57) **Abstract**

An anatomical knee joint prosthesis, comprising a femoral condyle (1), a tibial plateau support (3) and a tibial plateau pad (2) on side of which is fixed to the tibial plateau support (3) and the other side of which mates with the femoral condyle (1). The femoral condyle (1) comprises a lateral condyle (141), a medial condyle (142) and a trochlea (12) disposed between the medial condyle (142) and the lateral condyle (141). A connection line between a point of maximum curvature of the lateral condyle (141) and a point of maximum curvature of the medial condyle (142) has an angle of about 3°with a projection line of the connection line in the horizontal plane where the point of maximum curvature of the medial condyle (142) is located. The prosthesis has the effect of better fitting the anatomical form of a human body.

## Description

### TECHNICAL FIELD

The present application relates to a field of medical apparatus and instruments, and, in particular, to an anatomical knee joint prosthesis.

### BACKGROUND ART

For a knee joint, a conventional knee joint prosthesis generally includes a femoral condyle fixedly connected to a distal end of a femur, a tibial plateau support fixedly connected to a proximal end of a tibia, and a tibial plateau pad arranged between the femoral condyle and the tibial plateau support. The femoral condyle has a semi enclosed structure generally in a C-shape, and includes a lateral condyle, a medial condyle and a trochlea disposed at an opening groove between the lateral condyle and the medial condyle or recessed toward an interior of the femoral condyle. The maximum curvatures of longitudinal sections of the lateral condyle and the medial condyle are both positioned in a lower half, that is, a portion close to the tibial plateau pad. In a conventional design of the femoral condyle, the lateral condyle and the medial condyle are symmetrically arranged about the opening groove or the trochlea. Correspondingly, the tibial plateau pad cooperating with the femoral condyle is also designed to have a symmetric structure.

However, there are following deficiencies in the above technical solution: the knee joint prosthesis does not comply with human anatomical features, which easily leads to imbalanced soft tissue on two sides of knee joint in terms of flexion and extension, further leading to problems of an unstable j oint, an incorrect direction of force, a poor postoperative function, and failure to meet requirements for physiological exercises in terms of bending and unbending, influencing contact with the knee joint and a retention rate of prosthesis.

### SUMMARY

In the view of the deficiencies present in existing technologies, the present application provides an anatomical knee joint prosthesis being more conformal with human anatomical features.

The present application adopts the following technical solution to realize the above obj ects.

An anatomical knee joint prosthesis provided according to an aspect of the present application includes a femoral condyle, a tibial plateau support and a tibial plateau pad with one side thereof being fixed to the tibial plateau support and the other side thereof being in contact fit with the femoral condyle. The femoral condyle includes a lateral condyle, a medial condyle and a trochlea provided between the lateral condyle and the medial condyle. A connection line between a point where the lateral condyle has a maximum curvature and a point where the medial condyle has a maximum curvature has an angle of about 3° relative to a projection line of the connection line in a horizontal plane where the point where the medial condyle has the maximum curvature is located.

In the above technical solution, the structure of the lateral condyle and that of the medial condyle are asymmetric about the trochlea. The C-shaped longitudinal sections of the lateral condyle and the medial condyle have the same curvature. Two points where there is a maximum curvature are in a vertical plane parallel to a vertical plane of a human body, but the two points where there is a maximum curvature are located at different heights, so as to imitate the femoral condyle structure of a human body. Therefore, the anatomical knee joint prosthesis in the present application is more conformal with human anatomical features.

Preferably, the tibial plateau pad includes a medial plateau surface and a lateral plateau surface in contact cooperation with the medial condyle and the lateral condyle respectively. The medial plateau surface and the lateral plateau surface are both concaved surfaces recessed substantially at a center thereof. In addition, the medial plateau surface is further recessed relative to the lateral plateau surface.

Preferably, a column protruding upwards is provided at a substantially center of the tibial plateau pad, and an intercondylar groove for the column to penetrate through is defined in the trochlea of the femoral condyle.

Preferably, when the maximum curvature of the lateral condyle and the maximum curvature of the medial condyle are both located at a lowest point in a vertical direction, a connection line between a lower end of the femoral condyle and a lowest point of the intercondylar groove on an internal surface of the femoral condyle in a same vertical plane has an angle of about 15° relative to a horizontal plane.

Preferably, a plurality of fixing grooves perpendicular to a length direction of the femoral condyle are evenly defined in an internal wall of the femoral condyle.

Preferably, a vertical groove plate for surrounding the intercondylar groove is fixedly provided on the internal wall of the femoral condyle, and a bone cement groove is defined by the vertical groove plate and the internal wall of the femoral condyle.

Preferably, two ends of an interior of the intercondylar groove are both enclosed for imitating anterior and posterior cruciate ligaments.

Preferably, a top surface of the column is transited to a sidewall of the column in a smooth inclined curved surface.

Preferably, the tibial plateau support includes a support body for connecting to the tibial plateau pad and a tibial intramedullary column fixedly connected on a bottom surface of the support body.

Preferably, a portion of a sidewall of the support body is concaved inwardly to form a recess cooperating with a hollow portion of a tibial osteotomy surface, the tibial intramedullary column has an angle of about 93° relative to the bottom surface of the support body, and the tibial intramedullary column extends along a concaving direction of the sidewall of the support body.

Preferably, a plurality of granular protruded structures are evenly distributed on a lower surface of the support body.

Preferably, a locking groove is provided on a top surface of the support body, a sidewall of the support body and at least a portion of the locking groove form a snap-in groove structure extending toward an interior of the support body, a locking mechanism cooperating with the snap-in groove is fixedly provided on a bottom surface of the tibial plateau pad, and the locking mechanism is snap connected in the locking groove.

Thinned anterior condyle can reduce a pressure on the patella, so that the transition between the trochlea and the intercondylar groove is smoother, reducing clicking of the patella and pain of the anterior knee. In addition, an eversion of 3° is achieved by using a bionic thickness to reconstruct a 3° physiologic articular line, so that soft tissues on two sides reach a better balance to be more conformal with human anatomical features. The highly inflective posterior condyle of the femoral condyle is bended backwards by an angle of 15° to increase the attaching stability of the femur prosthesis and reducing the osteotomy volume of the posterior condyle of the femur. The posterior condyle in an obvious J-shaped curve increases the postroll of the tibial plateau during a deep knee flexion, reducing the impact between the femoral condyle and the tibial plateau and increasing the attaching stability of the femur prothesis. In addition, the bionic design of the tibial plateau pad has an internal recess and an external protrusion, reestablishing the physiological state of the knee joint meniscus, which is consistent with the human kinematics features. The tibial plateau is concaved at the front, so as to return to the normal starting AP (anterior posterior) position of the natural knee j oint, preventing the abnormal movement and simulating the normal knee joint movement, so as to maximize the depth of flexion.

In the above technical solution, the arrangement of the fixing groove is conductive to the adhering of the bone cement, increasing the connection strength between the femoral condyle and the bone cement, so that the femoral condyle can be stably connected to the human bone, increasing the retention rate and operation stability of prosthesis.

In the above technical solution, providing of the vertical groove plate can protect the intercondylar groove. When filling the bone cement in the bone cement groove, the vertical groove plate can reduce the possibility of the leaking of the bone cement leaks into the intercondylar groove and solidifying on the column to hinder a smooth movement of the femoral condyle relative to the column.

In the above technical solution, the enclosure of the front end of the intercondylar groove can serve the function of anterior cruciate ligament during extension and flexion. The front enclosure end of the intercondylar groove abuts against the column in an angel of 0°-20°, realizing the reestablishment of anterior cruciate ligament; and the rear enclosure end of the intercondylar groove abuts against the rear side of the column in an angel of 60°- 150°, realizing the reestablishment of posterior cruciate ligament, so as to imitate a normal knee joint. With two sides of the tibial plateau pad move backwards, the deep flexion is maximized.

In the above technical solution, when the user performs a knee bending action, the femoral condyle has a rotation and a movement relative to the tibial plateau pad. Since the front enclosure end of the intercondylar groove abuts against the column when the user stands uprights, the column has a friction with the front enclosure end of the intercondylar groove when knee flexion. The top surface of the column is transited to the sidewall of the column in the smooth inclined curved surface to reduce the friction between the column and the front enclosure end of the intercondylar groove, protecting the column and the femoral condyle and reducing the abnormal sound.

In the above technical solution, the design of a double wing reinforcing structure can not only increase the mechanical strength, but also improve the anti-rotation stability of the product.

In the above technical solution, the double wing reinforcing structure is configured close to each other and at an angle to each other, which is consistent with the human anatomical structure, and can better match with the human skeleton and tibia section, having a good matching, and further improving the anti-rotation stability.

In the above technical solution, the tibial intramedullary column extends towards the recess direction of the support body sidewall, and has an angle of 3°with the bottom plane of the support body, which is consistent with the human anatomical structure and has a higher matching degree with the medullary cavity of tibia, so that the tibia support is more stable after implantation into the medullary cavity.

In the above technical solution, the lower surface in a granular shape can increase the matching degree between the tibial plateau support and the tibia section after adding the bone cement, so that the covered bone is subjected to a uniform stress, reducing the shear stress and the axial load, and improving the operation stability of the tibial plateau support.

In the above technical solution, the locking groove and the locking mechanism can increase the connection strength and the connection stability between the tibial plateau pad and the tibial plateau support, extremely reducing the slight movement of the pad and the wear of the back portion.

In conclusion, the present application has at least one of the following beneficial effects:
Thinned anterior condyle can reduce the pressure on the patella, so that the transition between the trochlea and the intercondylar groove is smoother, reducing clicking of the patella and pain of the anterior knee. In addition, an eversion of 3° is achieved by using a bionic thickness, to reconstruct the 3° physiologic articular line, so that soft tissues on two sides reach a better balance to be more conformal with human anatomical features. The highly inflective posterior condyle of the femoral condyle is bended backwards by an angle of 15°, to increase the attaching stability of the femur prosthesis and reducing the osteotomy volume of the posterior condyle of the femur. The posterior condyle in an obvious J-shaped curve increases the postroll of the tibial plateau during a deep knee flexion, reducing the impact between the femoral condyle and the tibial plateau and increasing the attaching stability of the femur prothesis. In addition, the bionic design of the tibial plateau pad has an internal recess and an external protrusion, reestablishing the physiological state of the knee joint meniscus, which is consistent with the human kinematics features. The tibial plateau is concaved at the front, so as to return to the normal starting AP position of the natural knee joint, preventing the abnormal movement and simulating the normal knee joint movement, so as to maximize the depth of flexion;
the design of a double wing reinforcing structure can not only increase the mechanical strength, but also improve the anti-rotation stability of the product; and the double wing reinforcing structure is configured close to each other and at an angle to each other, which is consistent with the human anatomical structure, and can better match with the human skeleton and tibia section, having a good matching, and further improving the anti-rotation stability.

### BRIEF DESCRIPTION OF THE DRAWINGS

FIG. 1 is a structural schematic diagram of a femoral condyle, a tibial plateau pad and a tibial plateau support in a using state according to an embodiment.
FIG. 2 is a structural schematic diagram of femoral condyle according to an embodiment.
FIG.3 is an enlarged diagram of portion A in FIG.2 showing a locking structure and a locking groove.
FIG.4 is a schematic diagram showing an included angle between a medial condyle and a lateral condyle according to an embodiment.
FIG.5 is a schematic diagram showing a recess angle of a posterior condyle to an interior of a femoral condyle according to an embodiment.
FIG.6 is a schematic diagram showing a granular-shaped protrusion structure of a bottom surface of a support body according to an embodiment.

Listing of reference signs: 1 femoral condyle; 11 anterior condyle; 12 trochlea; 13 intercondylar groove; 14 posterior condyle; 141 lateral condyle; 142 medial condyle; 15 vertical groove plate; 16 fixing groove; 17 bone cement groove; 2 tibial plateau pad; 21 column; 22 bevel portion; 23 medial plateau surface; 24 lateral plateau surface; 25 locking mechanism; 26 external convex ridge; 27 upper clamping edge; 3 tibial plateau support; 31 support body; 32 tibial intramedullary column; 33 wing plate; 34 locking groove; 35 invagination groove; 36 lower clamping edge.

### DETAILED DESCRIPTION

The present application is further described in detail below in combination with the drawings. In the present application, the terms for indicating the direction such as "front", "back", "up" and "down" are consistent with the front, back, up and down directions of the knee joint prosthesis in the upright state after it is implanted into the human body. In the present application, a left leg knee joint prosthesis is taken as an example to describe.

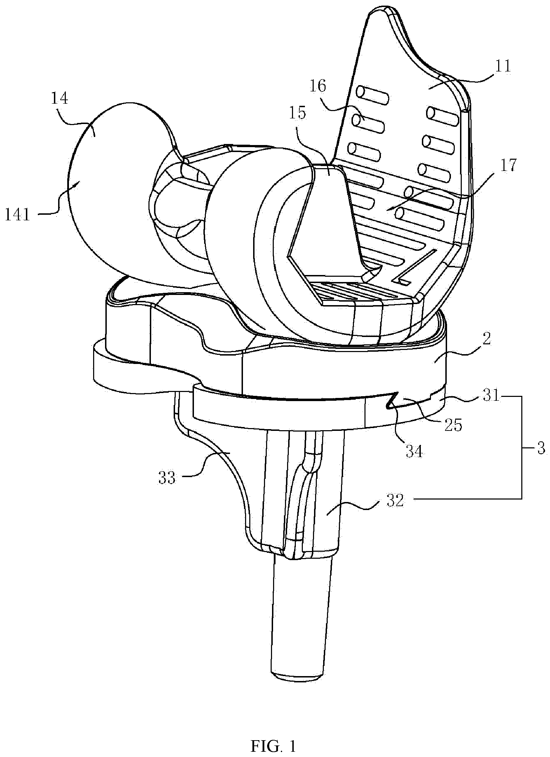

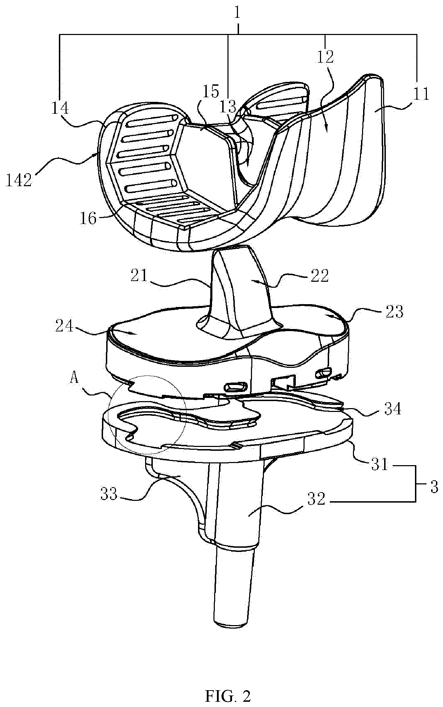

Referring to FIG.1 and FIG.2, an anatomical knee joint prosthesis disclosed in the present application includes a femoral condyle 1, a tibial plateau pad 2 and a tibial plateau support 3. Preferably, the tibial plateau pad 2 and the tibial plateau support 3 are fixedly connected to each other in a snapping manner. The femoral condyle 1 has a semi enclosed structure generally in a C-shape. The femoral condyle 1 includes a lateral condyle 141, a medial condyle 142, a trochlea 12 arranged along the contour thereof, and an intercondylar groove 13 for accommodating a column 21. An end of the trochlea 12 is called as an anterior condyle 11, and each side of the intercondylar groove 13 is called as a posterior condyle 14.

The intercondylar groove 13 penetrates through the femoral condyle 1, and the length direction of the intercondylar groove 13 is along the contour of the femoral condyle 1. Two ends of an interior of the intercondylar groove 13 are both enclosed to imitate the anterior and posterior cruciate ligaments, and for matching with the tibial plateau pad 2. A vertical groove plate 15 for surrounding the intercondylar groove 13 is fixedly provided on an internal wall of the femoral condyle 1. A bone cement groove 17 is defined by the vertical groove plate 15 and the internal wall of the femoral condyle 1. A plurality of fixing grooves 16 are provided on an internal wall of the bone cement groove 17, a length direction of the fixing groove 16 is perpendicular to a length direction of the intercondylar groove 13, and a plurality of fixing grooves 16 are distributed evenly along the contour of the femoral condyle 1.

The presence of the trochlea 12 makes an external wall of the femoral condyle 1 be recessed inwardly, so that the femoral condyle 1 has a V-shaped cross section. One end of the trochlea 12 is located at the anterior condyle 11 and the other end is located at the intercondylar groove 13. A thickness of the anterior condyle 11 is decreased from one end of the intercondylar groove 13 to the other end thereof.

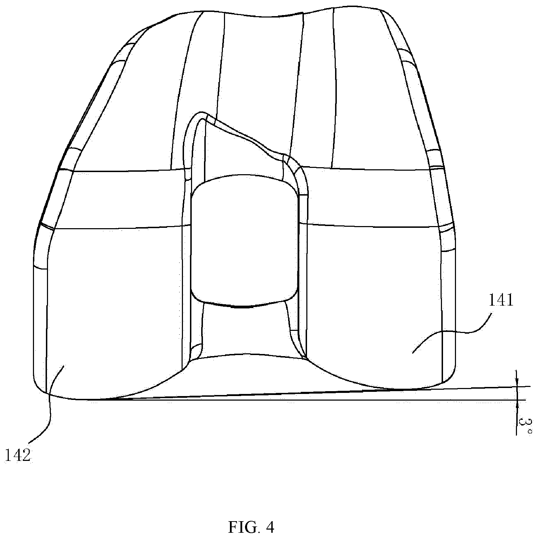

The posterior condyle 14 is divided into the lateral condyle 141 and the medial condyle 142 by the trochlea 12. The posterior condyle 14 at the lateral condyle 141 has a thickness of 6-8 mm, and the posterior condyle 14 at the medial condyle 142 has a thickness of 8-14 mm. During the operation of the knee joint prothesis in the present application, when the point where the the lateral condyle 141 has a maximum curvature and the point where the medial condyle 142 has a maximum curvature are both located at a lowest point in a vertical direction, a connection line between a point where the the lateral condyle 141 has the maximum curvature and a point where the medial condyle 142 has the maximum curvature has an angle of about 3° relative to a projection line of the connection line in the horizontal plane where the point where the medial condyle 142 has the maximum curvature is located (referring to FIG.4).

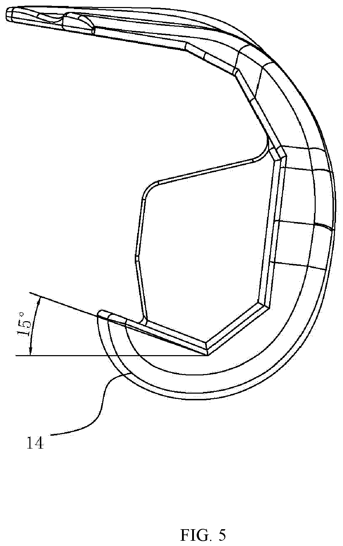

During the operation of the knee joint prothesis in the present application, when the maximum curvature of the lateral condyle 141 and the maximum curvature of the medial condyle 142 are both disposed at a lowest point in the vertical direction, a connection line between a lower end of the femoral condyle 1 and a lowest point of the intercondylar groove 13 on an internal surface of the femoral condyle 1 in a same vertical plane has an angle of about 15° with a horizontal plane, so that the contour of the entire posterior condyle 14 assumes a J-shape (referring to FIG.5).

A column 21 protruding into the intercondylar groove 13 is fixedly provided at a substantially center of the tibial plateau pad 2, so as to cooperate with the intercondylar groove 13 to function as anterior and posterior cruciate ligaments. Preferably, a top surface of the column 21 is transited to a sidewall (preferably one sidewall) of the column 21 in a smooth inclined curved surface, and a bevel portion 22 is formed on the sidewall of the column 21. The tibial plateau pad 2 is divided by the column 21 into a medial plateau surface 23 and a lateral plateau surface 24 cooperating with the medial condyle 142 and the lateral condyle 141 respectively. The medial plateau surface 23 and the lateral plateau surface 24 are both curved surface with a recessed center and raised edges. In addition, the medial plateau surface 23 is further recessed relative to the lateral plateau surface 24.

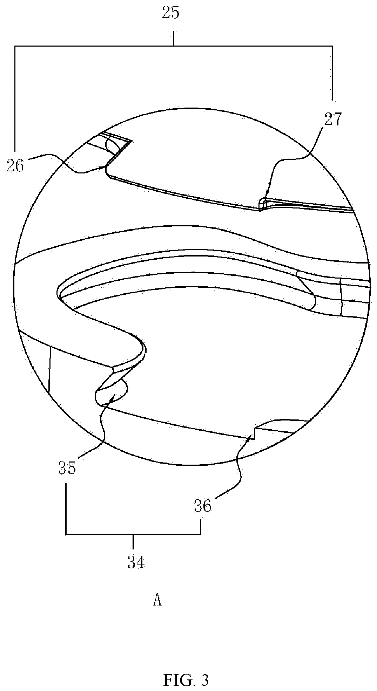

Referring to FIG.2 and FIG.3, the tibial plateau support 3 includes a support body 31 for connecting to the tibial plateau pad 2 and a tibial intramedullary column 32 fixedly connected on a bottom surface of the support body 31. A double wing reinforcing structure is fixedly provided on the tibial intramedullary column 32. In this embodiment, the double wing reinforcing structure includes two wing plates 33 in an included angle, which extend outwards along a radial direction of the sidewall of the tibial intramedullary column 32 and are fixedly provided on the bottom surface of the support body 31. The sidewall of each wing plate 33 is fixedly connected to the tibial intramedullary column 32. A portion of the support body 31 sidewall is concaved inwardly to form a recess matching with a hollow portion of a tibial osteotomy surface. The recess is used for reducing the cost and obtaining a largest cover for the tibial osteotomy surface, so that the pressure is evenly distributed on the approximal end of the tibia, reducing the compression of the tibial bracket on the surrounding soft tissues, reducing the pain after the operation and facilitating the deep bending and crouching.

A locking groove 34 is provided on the top surface of the support body 31, in which the circumference of the support body 31 is extended and the extending portion recesses downwards to form the locking groove 34. A locking mechanism 25 for matching with the locking groove 34 is fixedly provided on the bottom surface of the tibial plateau pad 2. The locking groove 34 includes an invagination groove 35 in a clamping groove structure concaved inwards formed by the support body 31 sidewall and at least a portion of the locking groove 34, and a lower clamping edge 36 positioned at two ends of the invagination groove 35. The lower clamping edge 36 and the invagination groove 25 form a plane trough structure for accommodating the locking mechanism 25. The locking mechanism 25 includes an external convex ridge 26 for matching with the invagination groove 35 formed on the bottom surface of the tibial plateau pad 2 and an upper clamping edge 27 for matching with the lower clamping edge 36. When the external convex ridge 26 is inserted in the invagination groove 35, the upper clamping edge 27 abuts against the lower clamping edge 36.

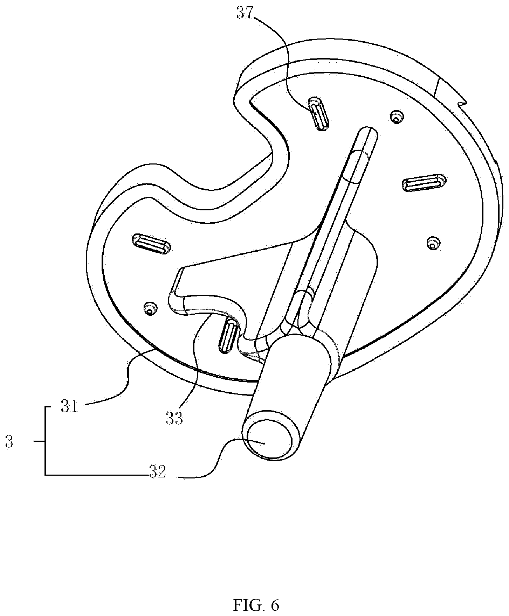

Referring to FIG.6, a plurality of protruding structures in a granular shape are evenly distributed on a lower surface of the support body 31. The protruding structure in a granular shape can be configured as hemispherical or semi cylindrical.

The above are the preferred embodiments of the present application, which are not intended to limit the protection scope of the present application. Therefore, all equivalent changes made according to the structure, shape and principle of the present application should be covered within the protection scope of the present application.

## Claims

1. An anatomical knee joint prosthesis comprising a femoral condyle (1), a tibial plateau support (3), and a tibial plateau pad (2) with one side thereof being fixed to the tibial plateau support (3) and the other side thereof being in contact fit with the femoral condyle (1), wherein the femoral condyle (1) comprises a lateral condyle (141), a medial condyle (142) and a trochlea (12) provided between the lateral condyle (141) and the medial condyle (142), **characterized in that**, a connection line between a point where the lateral condyle (141) has a maximum curvature and a point where the medial condyle (142) has a maximum curvature has an angle of about 3° relative to a projection line of the connection line in a horizontal plane in which the point where the medial condyle (142) has the maximum curvature is located.

2. The anatomical knee joint prosthesis according to claim 1, **characterized in that**, the tibial plateau pad (2) comprises a medial plateau surface (23) and a lateral plateau surface (24) in contact fit with the medial condyle (142) and the lateral condyle (141), respectively; and the medial plateau surface (23) and the lateral plateau surface (24) are both concaved surfaces recessed substantially at a center thereof, and the medial plateau surface (23) is further recessed relative to the lateral plateau surface (24).

3. The anatomical knee joint prosthesis according to claim 1, **characterized in that**, a column (21) protruding upwards is provided at a substantially center of the tibial plateau pad (2), and an intercondylar groove (13) for the column (21) to penetrate through is defined in the trochlea (12) of the femoral condyle (1).

4. The anatomical knee joint prosthesis according to claim 1, **characterized in that**, when the maximum curvature of the lateral condyle (141) and the maximum curvature of the medial condyle (142) are both located at a lowest point in a vertical direction, a connection line between a lower end of the femoral condyle (1) and a lowest point of the intercondylar groove (13) on an internal surface of the femoral condyle (1) in a same vertical plane has an angle of about 15° relative to a horizontal plane.

5. The anatomical knee joint prosthesis according to claim 1, **characterized in that**, a plurality of fixing grooves (16) perpendicular to a length direction of the femoral condyle (1) are evenly defined in an internal wall of the femoral condyle (1).

6. The anatomical knee joint prosthesis according to claim 1, **characterized in that**, a vertical groove plate (15) for surrounding the intercondylar groove (13) is fixedly provided on the internal wall of the femoral condyle (1), and a bone cement groove (17) is defined by the vertical groove plate (15) and the internal wall of the femoral condyle (1).

7. The anatomical knee joint prosthesis according to claim 1, **characterized in that**, two ends of an interior of the intercondylar groove (13) are both enclosed for imitating anterior and posterior cruciate ligaments.

8. The anatomical knee joint prosthesis according to claim 1, **characterized in that**, a top surface of the column (21) is transited to a sidewall of the column (21) in a smooth inclined curved surface.

9. The anatomical knee joint prosthesis according to claim 1, **characterized in that**, the tibial plateau support (3) comprises a support body (31) for connecting to the tibial plateau pad (2) and a tibial intramedullary column (32) fixedly connected on a bottom surface of the support body (31).

10. The anatomical knee joint prosthesis according to claim 9, **characterized in that**, a portion of a sidewall of the support body (31) is concaved inwardly to form a recess cooperating with a hollow portion of a tibial osteotomy surface, the tibial intramedullary column (32) has an angle of about 93° relative to a bottom surface of the support body (31), and the tibial intramedullary column (32) extends along a concaving direction of the sidewall of the support body (31).

11. The anatomical knee joint prosthesis according to claim 1, **characterized in that**, a plurality of granular protruded structures are evenly distributed on a lower surface of the support body (31).

12. The anatomical knee joint prosthesis according to claim 1, **characterized in that**, a locking groove (34) is provided on a top surface of the support body (31), a sidewall of the support body (31) and at least a portion of the locking groove (34) form a snap-in groove structure extending toward an interior of the support body (31), a locking mechanism (25) cooperating with the snap-in groove (34) is fixedly provided on a bottom surface of the tibial plateau pad (2), and the locking mechanism (25) is snap connected in the locking groove (34).
